# EUROPEAN PATENT SPECIFICATION

(11) **EP 1 242 023 B1**
(45) Date of publication and mention of the grant of the patent: **23.01.2008**
(21) Application number: 99962214.5
(22) Date of filing: 03.12.1999
(51) Int. Cl.: A61F 13/15

(54) **INTERMEDIATE ABSORBENT STRUCTURE WITH INTEGRATED PARTICLE BARRIER**
SAUGFÄHIGE STRUKTUR MIT INTEGRIERTEN PARTIKEL-SPERRSCHICHTEN
STRUCTURE ABSORBANTE INTERMEDIAIRE AVEC BARRIERE ANTI-PARTICULES INTEGREE

(43) Date of publication of application: 25.09.2002
(73) Proprietor: KORMA S.p.A., 20050 Peregallo di Lesmo (IT)
(72) Inventor: GERLACH, Herbert, I-24010 Ponteranca (IT)
(74) Representative: Maxton Langmaack & Partner
(86) International application number: PCT/EP1999/009490
(87) International publication number: WO 2001/039707

(56) References cited:
- EP-A- 0 627 210
- EP-A- 0 689 818
- EP-A- 0 819 414
- EP-A- 0 846 455
- WO-A-95/03019
- WO-A-97/07761
- GB-A- 2 269 109
- US-A- 4 655 757
- US-A- 5 334 177
- US-A- 5 364 382

## Description

This invention relates to intermediate absorbent structures comprising an upper layer that is liquid permeable, a lower layer and an intermediate layer arranged between said upper layer and said lower layer, said intermediate layer comprising absorbent material preferably comprising superabsorbent polymer in the form of particles or small fibers dispersed between said upper layer and said lower layer, and optionally comprising a thermoplastic bonding agent, wherein said absorbent core is produced as part of a continuous web of absorbent cores, wherein the width of said continuous web is approximately equivalent to the width of a multiple number of cores, wherein said absorbent material is deposited in a defined pattern, as defined in the preamble of patent claim 1.

Intermediate absorbent structures as defined in the preamble of claim 1 are known from EP 0 7D8 628 B1. Intermediate absorbent structures of this type serve to provide, by means of an off-line process, intermediate multilayer absorbent structures and products, incorporating powders or fibers or granules of absorbent material, which products may also be of small size, with a maximum design flexibility and without appreciable additional costs from modifying the shape or the structure of the absorbent article.

Although intermediate absorbent structures known from EP 0 708 628 B1 do have a number of advantages, it has, however, being found that they do have also some disadvantages and shortcomings.

For better understanding of the invention, as used herein, the term "absorbent structure" refers to disposable absorbent articles for personal or medical care, such as catamenial devices, incontinence devices, diapers, dressings and the like. They may also be used for household or industrial applications, such as cleaning devices. Intermediate absorbent structures are manufactured in the form of a continuous web that can be converted into absorbent articles. In final absorbent articles, said absorbent structures are often covered by an external covering material.

Common elements in disposable absorbent articles such as catamenial devices, incontinence devices, disposable diapers, dressings and the like include a fluid permeable surface material to be placed adjacent to the site of fluid discharge, a fluid storage core, and a fluid impervious surface external to the storage core for fluid containment. A variety of forms and configurations of absorbent materials in storage cores, often including fibrous materials, in absorbent articles are known in the art. Storage cores typically include superabsorbent materials in the form of particles, powder, granules or fibers due to the excellent absorption and storage capabilities of these materials. Typically the size of the particles in a powder material Is in a range of 40 to 1400 µm, whereas above such upper limit, they are more appropriately referred to as granules. For the purpose of this application, powder and granules will be included in the term particles. Other particles or fibers with a function such as odor absorption or treatment may also be present in the core.

As the core of an absorbent article is intended to permit liquid entry and storage, the arrangement of materials inside the core is designed to allow fluid flow to the absorbent particles or fibers. A difficulty in using such absorbent materials in a typical absorbent article is the migration and loss of absorbent material. Migration and inappropriate local concentration of absorbent material can lead to diminished article effectiveness. Penetration of superabsorbent material to an external layer of a final article can have a particularly negative impact on both the esthetic aspects of a finished article, for example on the outward-facing portion of a diaper or a wound dressing, and on the containment function of the article. Lost absorbent material can result in extra manufacturing and handling measures to cope with and recuperate lost material, the need to overdose the quantity of absorbent material to compensate for loss, a negative esthetic appearance of the article and diminished effectiveness of the article. This problem is particularly relevant with regard to intermediate absorbent products that are intended to be included in final absorbent articles. Said intermediate products are commonly manufactured as continuous webs to be rolled, and later separated into individual intermediate articles to be used as at least part of the storage core of final articles.

Due to the risk of migration and loss, said absorbent particles or fibers require substantial gluing or creation of adhesion points within the core. However, glues and adhesives are each known to reduce the absorption capability of absorbent materials. Common approaches to solving this dilemma include the addition of an excessive amount of absorbent material to the core, and decreasing the amount of glue or adhesive used in the core to a level where it does not interfere with the absorbent materials. The latter approach is preferable as it requires a lower quantity of raw materials, however an obvious result of a decrease in the quantity of adhesive is an increase in the quantity of loose absorbent material.

A number of solutions to the problem of absorbent material loss through the longitudinal and transverse edges of absorbent cores have been disclosed, particularly with reference to intermediate absorbent articles, as for example in European Pat. Appl. No. 846455 to Corzani et al. Particle loss through the topsheet of final articles has been addressed in, for example, European Pat. Appl. No. 749736 to Divo et al. or European Pat. Appl. No. 682927 to Bitowft et al. Such a topsheet may comprise the core supporting material or upper core material used in the manufacture of intermediate articles. More complex approaches to particle loss from the entire article that involve wrapping the absorbent core in a particle containment material have been disclosed in, for example, US Pat. No. 5,458,592 to O'Brien et al. or WO 97/07761 to Veith et al as well as in WO 95/03019. From WO 97/07761 it is known to manufacture an intermediate absorbent structure consisting of an upper layer, a lower layer and an intermediate layer whereby the lower layer is impervious to the penetration of particles of absorbent material.

It is an object of the present invention to provide an intermediate absorbent structure which overcomes the aforementioned shortcomings and disadvantages; which may be produced at minimum costs and which supports and improves the characteristics of the final absorbent product.

These objects are accomplished by the invention thanks to the features of the characterising portion of claim 1. Other advantages and practical features are the subjects of the dependent claims.

According to the invention, an intermediate absorbent structure comprises an upper layer that is liquid permeable, a lower layer and an intermediate layer. The intermediate layer is arranged between the upper layer and the lower layer. The intermediate layer comprises absorbent material between the upper layer and the lower layer, wherein an absorbent core is produced as part of a continuous web of absorbent cores in a multiple number of cores along a width of the web comprising the upper layer (2), the lower layer (3) and the intermediate layer (4), wherein the absorbent material is deposited in a defined pattern, and wherein the lower layer is impervious to the penetration of particles of absorbent material or odor regulating material in the form of particles or small fibers. The lower layer has a mean pore size in the range of 5-25 µm. A width of the continuous web is equivalent, respectively approximately equivalent to the width of a multiple number of cores, wherein at least 70 % of the pores of the lower layer have a diameter in the range 5-15 µm, and wherein said lower layer has an air permeability less than 1000 m/sec at 196 Pa, tensile strength in the machine direction of at least 20 N/5 cm and a tensile strength in the cross direction of at least 5 N/5 cm and wherein the lower layer and the upper layer are bonded together by adhesive strips.

In other words, the problems of the prior art are solved by a lower layer consisting of a material which can't be penetrated by particles of the absorbent material. This material may be a synthetic film, a nonwoven or a film-nonwoven composite.

Surprisingly, a number of advantages may be achieved by using materials as defined in the characterising portion of claim 1 in the lower layer. As the lower layer prevents penetration of particles of the absorbent material, the backsheet material or an external covering of the final product may now be made of less expensive materials and of materials having better characteristics for the user, respectively.

Another advantage of the invention is the avoidance of loss and migration of the expensive absorbent material during the off-line production of the intermediate absorbent structure according to the invention.

This type of barrier also has the advantage of being fluid permeable, but nonabsorbing, to permit passage and absorption of liquids that bypass the absorbent core upon discharge, and thereby reducing fluid contact with the backsheet or external covering of the final article.

Other objects and features of the present invention will become apparent from the depending claims and from the following detailed description describing a preferred embodiment of the invention. It should be understood, however, that the description is not a definition of the limits of the invention.

A layered intermediate absorbent structure comprising a breathable barrier composite according to the invention will be produced according to the teachings of EP 0 708 628 B1.

At least a lower layer of the absorbent structure according to the invention is impervious to the penetration of particles of absorbent material or odor absorbing material in the form of particles or small fibers. Said layer has a mean pore size in the range 5 - 25 µm, wherein at least 70 % of the pores have a diameter in the range 5 - 15 µm. Pore size can be measured using a standard light microscope. Preferably at least 10 random areas of dimensions 1 mm x 1 mm are selected at random from a given sample for measurement. The diameters of the pores within the given areas are measured using a magnification of 80x and a suitable image analysis system, for example a Quantimet Image Analysis System. fication of 80x and a suitable image analysis system, for example a Quantimet Image Analysis System.

Said lower layer has an air permeability less than 1000 m/s at 196 Pa, as measured according to Edana RTM 140.199 for Nonwovens Air Permeability amended such that the pressure drop is 196 Pa (as in Edana RTM 140.1-81) instead of 200 Pa.

The lower layer has a tensile strength in the machine direction of at least 20 N/5 cm and a tensile strength in the cross direction of at least 5 N/5 cm measured according to Edana RTM 20.2-89.

The intermediate absorbent structure or absorbent core is produced as part of a continuous web of cores as described in EP 0 708 628 B1.

The sequence of intermediate structure formation generally proceeds starting with a web of either the so-called upper layer, or the lower layer used as a supporting layer for the depositing of a layer comprising absorbent material. In one embodiment of the invention the intermediate absorbent article may comprise more than one liquid permeable layer and more than one layer of absorbent material.

The liquid permeable upper layer is selected from the group woven fabric, nonwoven fabric, synthetic film, tissue paper, air-laid paper, air-laid composites, and composites of fine fibers and continuous filaments. In one embodiment, the upper layer might comprise cellulose fibers with a basis weight in the range 20-1000g/m², preferably in the range 20-500 g/m². Basis weight can be evaluated according to the Edana RTM 40.3-90 for Nonwovens Mass per Unit Area. Alternatively, the material of the upper layer may comprise 50-100 % cellulose. Furthermore, the upper layer may comprise at least 1% synthetic materials selected from the group polyolefins, polypropylene, polyethylene, polyamides, polyesters and polyetheresters. In a preferred embodiment the upper layer is a layer of air-laid paper. Said layer may be formed in-line and said layer may include stiffening agents, such as for example chemically stiffened pulp, for resiliency and form maintenance.

The layer of absorbent material comprises material selected from the group absorbent gelling material, absorbent particles, superabsorbent particles, absorbent fibers and superabsorbent fibers. The absorbent material can be either a single absorbent material or a blend of absorbent materials, comprising material that is capable of turning into a gel upon being wetted, and thus retaining large amounts of liquids with respect to its own original known absorbent materials can be used, both in powder and fiber form. A variety of such materials are known to the art.

In a preferred embodiment, said layer comprises superabsorbent gelling material in the form of particles. In addition, the absorbent material may comprise material with an odor absorbing or odor controlling function such as, for example, zeolites, silica, carbon or pH-regulating material. The basis weight of the deposited absorbent material, measured in the product and with respect to the deposited areas, is preferably from 10 to 1000 g/m².

Said absorbent material may be deposited as a single layer or as multiple layers between the upper and lower layers of the article. Alternative to the deposition of absorbent material in between containing layers, an absorbent composite may be formed by the mixing of absorbent material with accompanying supporting fibers comprising natural or synthetic fibers, and optionally including bicomponent binder fibers, as may be formed using conventional air laid technology.

In a preferred embodiment of the invention, the lower layer comprises a composite of fine fibers and continuous filaments with a total basis weight of fine fibers in the range 2 to 40 g/m². and a total basis weight of continuous filaments in the range 8 to 48 g/m². The production of such composites is well described in the art. Most preferably, said composite of fine fibers and continuous filaments comprises a spunbond-meltblown composite with a spunbond layer with a basis weight in the range 4 to 24 g/m² and a meltblown layer with a basis weight in the range 2 to 20 g/m². Alternative composite compositions are a spunbond layer with a basis weight in the range 10 to 20 g/m² and a meltblown layer with a basis weight in the range 2 to 12 g/m², or a spunbond layer with a basis weight in the range 2 to 9 g/m² and a meltblown layer with a basis weight in the range 1 to 2 g/m². In an additional embodiment, the composite of fine fibers and continuous filaments comprises at least one spunbond layer and at least one meltblown layer. The material of the lower layer is selected from the group polyolefins, polypropylene, polyethylene, polyamides, polyesters and polyetheresters, most preferably from the group polyethylene and polypropylene.

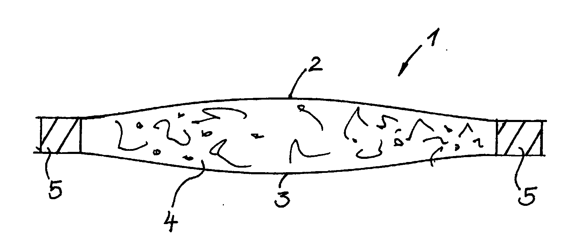

The formation of simple spunbond-meltblown composites is known to the art. In a preferred embodiment, the spunbond-meltblown composite of the lower layer is manufactured using a forming line that comprises at least one station for the melt extrusion of continuous filaments and at least one station for the melt extrusion of fine fibers. The line preferably comprises one continuous filament station and one fine fiber station, the fine fiber station preferably lying downline from the continuous filament station. In the case of a simple two-layer composite, the continuous filaments are formed by the extrusion of molten polymer through spinnerets followed by the drawing of the filaments and the depositing of the filaments in a random fashion on a moving belt. Fine fibers produced by the meltblown technique are then deposited onto the continuous filament layer in a random fashion. Downline of the extrusion stations, the composite is intermittently bonded using a heated calender. Rolling and possibly slitting of the composite may follow bonding.

The material forming the adhesive strips can be a so called hot melt comprising various materials, such as APP, SBS. SEBS, SIS, EVA and the like, or a cold glue, such as a dispersion of various materials, e. g. SBS, natural rubber and the like, or even a solvent-based or a two-component adhesive system. Furthermore, the material may be capable of crosslinking to form specific, permanent chemical bonds with the various layers. The amount of adhesive is a function of the type of adhesive used, however it is generally between 0.2 and 20 g/m.

According to an embodiment the upper layer and the intermediate layer of absorbent material are bonded together thermally, optionally with the use of steam.

According to a further embodiment the lower layer and the intermediate layer of absorbent material are bonded together at discrete points, optionally using a thermally activated thermoplastic polymer or a pressure sensitive adhesive.

The rolled intermediate product webs are preferably unrolled, slit into continuous webs the width of a single absorbent core, and festooned. The web is preferably festooned into firm packaging, but may also be festooned into soft, compressible packaging or onto pallets. Alternatively, the web may be festooned in-line with or without prior slitting.

According to an embodiment of the manufacture of an intermediate absorbent structure a step of longitudinally and/or longitudinally slitting the web of cores is included. Another step comprises a trimming of the web.

According to an embodiment concerning a manufacturing of an intermediate absorbent product the following steps are comprised:
a) optionally preparing air laid paper or air laid composite material to be used as a web-like structure,
b) depositing absorbent material, odor controlling material, and optionally thermoplastic binder, onto a web-like substrate,
c) bonding said absorbent material and odor controlling material to the substrate, preferably with the use of heat,
d) depositing adhesive material at least in longitudinal strips, preferably along the intended core perimeters, onto the substrate,
e) applying a further web-like sheet on top of the assembly prepared in steps (a) to (c),
f) optionally repeating one or more of the steps (a) to (e) at least once,
g) optionally longitudinally slitting the web-like composite so obtained,
h) and festooning the slit or un-slit web.

An illustrative embodiment of the invention is shown in the attached drawing, which is an enlarged cross-section of an example of the invention.

In the drawing is shown a non-limiting example of an intermediate absorbent structure 1 comprising an upper layer 2 and a lower layer 3 and an intermediate layer 4. Said intermediate layer 4 is closely enveloped in between the upper layer 2 and the lower layer 3.

Upper layer 2 and lower layer 3 are bonded together by adhesive strips 5.

The physical properties of lower layer 3 are differing from the physical properties of upper layer 2, as described above.

Those skilled in the art will recognize that the absorbent core with integrated particle barrier of this invention may be used in a variety of final absorbent articles comprising particles or small fibers, such as for example catamenial pads, diapers, incontinence devices, wound dressings and in other applications not disclosed in the preceding description.

## Claims

1. An intermediate absorbent structure (1) comprising
a) an upper layer (2) that is liquid permeable,
b) a lower layer (3) and
c) an intermediate layer (4) arranged between said upper layer (2) and said lower layer, said intermediate layer comprising absorbent material between said upper layer and said lower layer, wherein an absorbent core is produced as part of a continuous web of absorbent cores in a multiple number of cores along a width of the web comprising the upper layer (2), the lower layer (3) and the intermediate layer (4), wherein said absorbent material is deposited in a defined pattern, and wherein the lower layer is impervious to the penetration of particles of absorbent material or odor regulating material in the form of particles or small fibers, said lower layer having a mean pore size in the range of 5-25 µm,
**characterized in that**
a width of said continuous web is equivalent to the width of a multiple number of cores and at least 70 % of the pores of said lower layer have a diameter in the range of 5-15 µm, and **in that** said lower layer has an air permeability less than 1000 m/s at 196 Pa, a tensile strength in the machine direction of at least 20 N/5 cm and a tensile strength in the cross direction of at least 5 N/5 cm and wherein said lower layer and said upper layer are bonded together by adhesive strips.

2. The structure (1) of claim 1 comprising individual cores of width in the range 20-2000 mm.

3. The structure (1) of claim 1 comprising the upper layer (2) selected from the group of woven fabric, nonwoven fabric, synthetic film, tissue paper, air-laid paper, air-laid composites, and composites of fine fibers and continuous filaments.

4. The structure (1) of claim 1 comprising the lower layer with a hydrophilic additive.

5. The structure (1) of claim 1 comprising a composite of fine fibers and continuous filaments.

6. The structure (1) of claim 5 comprising the composite of fine fibers and continuous filaments with a basis weight of fine fibers in the range 2 to 40 g/m², and a total basis weight of continuous filaments in the range 8 to 48 g/m².

7. The structure (1) of claim 5 with the composite of fine fibers and continuous filaments comprising a spunbond-meltblown composite.

8. The structure (1) of claim 5 wherein the basis weight of continuous filaments is about 4 - 24 g/m² and the basis weight of fine fibers is about 2 - 20 g/m².

9. The structure (1) of claim 5 comprising a spunbond layer with a basis weight in the range 10 to 20 g/m² and a meltblown layer with a basis weight in the range 2 to 12 g/m².

10. The structure (1) of claim 5 comprising a spunbond layer with a basis weight in the range 2 to 9 g/m² and a meltblown layer with a basis weight in the range 1 to 2 g/m².

11. The structure (1) of claim 5 comprising at least one spunbond layer and at least one meltblown layer.

12. The structure (1) of claim 1 including an intermediate layer (4) of absorbent material comprising absorbent material selected from the group absorbent gelling material, superabsorbent particles and superabsorbent fibers.

13. The structure (1) of claim 1 including an intermediate layer (4) of absorbent material comprising odor regulating material selected from the group zeolites, silica, clay, carbon and pH regulating compounds.

14. The structure (1) of claim 1 including an upper layer (2) and the intermediate layer (4) of absorbent material which are bonded together thermally.

15. The structure (1) of claim 1 wherein the lower layer (3) and the intermediate layer (4) of absorbent material are bonded together at discrete points.

16. The structure (1) of claim 15 wherein a thermally activated thermoplastic polymer selected from the group polyolefins, polypropylene, polyethylene, and ethylene vinyl acetate is used to bond the layers.

17. The structure (1) of claim 1 comprising more than one liquid permeable layer and more than one layer of absorbent material.

18. The structure (1) of claim 1 wherein the upper layer (2) comprises cellulose fibers with a basis weight in the range 20 - 1000 g/m².

19. The structure (1) of claim 1 wherein a material of the upper layer (2) comprises 50 - 100 % cellulose.

20. The structure (1) of claim 1 wherein the upper layer (2) comprises at least 1% synthetic materials selected from the group polyolefins, polypropylene, polyethylene, polyamides, polyesters and polyetheresters.

21. The structure (1) of claim 1 wherein the material of the lower layer (3) is selected from the group polyolefins, polypropylene, polyethylene, polyamides, polyesters and polyetheresters.

22. A final absorbent article comprising an absorbent core made from an intermediate absorbent structure of claim 1, wherein the absorbent article comprises the absorbent core with an integrated barrier.

## Patentansprüche

1. Eine dazwischen liegende absorbierende Struktur (1) aufweisend
a) eine obere Lage (2), die flüssigkeitsdurchlässig ist,
b) eine untere Lage (3) und
c) eine dazwischen liegende Lage (4), die zwischen der oberen Lage (2) und der unteren Lage angeordnet ist, die dazwischen angeordnete Lage weist ein Absorbentmaterial zwischen der oberen Lage und der unteren Lage auf, wobei ein absorbierender Kern produziert ist als Teil eines kontinuierlichen Stoffes von Absorbentkernen in einer vielzahligen Anzahl von Kernen entlang einer Breite des Stoffes, aufweisend die obere Lage (2), die untere Lage (3) und die dazwischen angeordnete Lage (4), wobei das Absorbentmaterial in einem definierten Muster deponiert ist, und wobei die untere Lage gegenüber einer Penetrierung von Partikeln des Absorbentmaterials oder eines Geruch regulierenden Materials in Form von Partikeln oder schmalen Fasern undurchlässig ist, die untere Lage hat eine mittlere Porengröße in dem Bereich von 5 bis 25 µm,
**dadurch gekennzeichnet, dass**
eine Breite des kontinuierlichen Stoffes entsprechend zu der Breite der vielzähligen Anzahl an Kernen ist und zumindest 70 % der Poren der unteren Lage einen Durchmesser in dem Bereich von 5 bis 15 µm aufweisen, und dass die untere Lage eine Luftdurchlässigkeit von weniger als 1000 m/s bei 196 Pa hat, eine Reißfestigkeit in Maschinenrichtung von zumindest 20 N/5 cm und eine Reißfestigkeit in Querrichtung von zumindest 5 N/5 cm und wobei die untere Lage und die obere Lage miteinander durch adhäsive Streifen verbunden sind.

2. Die Struktur (1) aus Anspruch 1, aufweisend individuelle Kerne einer Breite in dem Bereich von 20 bis 2000 mm.

3. Die Struktur (1) aus Anspruch 1, aufweisend die obere Lage (2) ausgewählt aus der Gruppe aus gewebten Stoffen, nicht gewebten Stoffen, synthetischen Film, Tissue-Papier, Airlaid-Papier, Airlaid-Composites und Composites aus feinen Fasern und kontinuierlichen Filamenten.

4. Die Struktur (1) aus Anspruch 1, aufweisend die untere Lage mit einem hydrophilen Additiv.

5. Die Struktur (1) aus Anspruch 1, aufweisend ein Composite aus feinen Fasern und kontinuierlichen Filamenten.

6. Die Struktur (1) aus Anspruch 5, aufweisend das Composite aus feinen Fasern und kontinuierlichen Filamenten mit einem Basisgewicht an feinen Fasern in dem Bereich von 2 bis 40 g/m² und einem vollständigen Basisgewicht an kontinuierlichen Filamenten in dem Bereich von 8 bis 48 g/m².

7. Die Struktur (1) aus Anspruch 5 mit dem Composite aus feinen Fasern und kontinuierlichen Filamenten, aufweisend ein Spunbond-Meltblown-Composite.

8. Die Struktur (1) aus Anspruch 5, wobei das Basisgewicht der kontinuierlichen Filamente ungefähr 4 bis 24 g/m² ist und das Basisgewicht an feinen Fasern ist ungefähr 2 bis 20 g/m².

9. Die Struktur (1) aus Anspruch 5, aufweisend eine Spunbond-Lage mit einem Basisgewicht in dem Bereich von 10 bis 20 g/m² und einer Meltblown-Lage mit einem Basisgewicht in dem Bereich von 2 bis 12 g/m².

10. Die Struktur (1) aus Anspruch 5, aufweisend eine Spunbond-Lage mit einem Basisgewicht in dem Bereich von 2 bis 9 g/m² und einer Meltblown-Lage mit einem Basisgewicht in dem Bereich von 1 bis 2 g/m².

11. Die Struktur (1) aus Anspruch 5, aufweisend zumindest eine Spunbond-Lage und zumindest eine Meltblown-Lage.

12. Die Struktur (1) aus Anspruch 1, enthaltend eine Zwischenlage (4) aus Absorbentmaterial aufweisend Absorbentmaterial ausgewählt aus der Gruppe absorbierendes Gelmaterial, super-absorbierende Partikel (SAP) und super absorbierende Fasern (SAF).

13. Die Struktur (1) aus Anspruch 1, enthaltend eine Zwischenlage (4) aus absorbierendem Material aufweisend geruchsregulierendes Material ausgewählt aus der Gruppe Zeolite, Silikate, Kreide, Kohlenstoff und pH-regulierende Mischungen.

14. Die Struktur (1) aus Anspruch 1, enthaltend eine obere Lage (2) und die Zwischenlage (4) aus absorbierendem Material, die zusammen thermisch verbunden sind.

15. Die Struktur (1) aus Anspruch 1, wobei die untere Lage (3) und die Zwischenlage (4) aus absorbierendem Material miteinander an diskreten Punkten verbunden sind.

16. Die Struktur (1) aus Anspruch 15, wobei ein thermisch aktiviertes thermoplastisches Polymer ausgewählt aus der Gruppe Polyolefine, Polypropylene, Polyethylene, und Ethylenvinylacetate genutzt wird, die Lagen zu verbinden.

17. Die Struktur (1) aus Anspruch 1, aufweisend mehr als eine flüssigkeitsdurchlässige Lage und mehr als eine Lage aus absorbierendem Material.

18. Die Struktur (1) aus Anspruch 1, wobei die obere Lage (2) Zellulosefasern mit einem Basisgewicht in dem Bereich 20 bis 1000 g/m² aufweist.

19. Die Struktur (1) aus Anspruch 1, wobei ein Material der oberen Lage (2) 50 bis 100 % Zellulose aufweist.

20. Die Struktur (1) aus Anspruch 1, wobei die obere Lage (2) zumindest 1 % synthetische Materialien ausgewählt aus der Gruppe Polyolefine, Polypropylene, Polyethylene, Polyamide, Polyester und Polyetherester aufweist.

21. Die Struktur (1) aus Anspruch 1, wobei das Material der unteren Lage (3) aus der Gruppe Polyolefine, Polypropylene, Polyethylene, Polyamide, Polyester und Polyetherester ausgewählt ist.

22. Ein absorbierender Endartikel aufweisend einen Absorbentkern hergestellt aus einer dazwischen angeordneten Absorbentstruktur gemäß Anspruch 1, wobei der absorbierende Artikel den Absorbentkern mit einer integrierten Barriere aufweist.

## Revendications

1. Structure absorbante intermédiaire (1), comprenant :
a) une couche supérieure (2) qui est perméable aux liquides,
b) une couche inférieure (3) et
c) une couche intermédiaire (4) agencée entre ladite couche supérieure (2) et ladite couche inférieure, ladite couche intermédiaire comprenant un matériau absorbant entre ladite couche supérieure et ladite couche inférieure, une âme absorbant étant produit en tant que partie d'une bande continue d'âmes absorbantes dans un nombre multiple d'âmes le long d'une largeur de la bande comprenant la couche supérieure (2), la couche inférieure (3) et la couche intermédiaire (4), ledit matériau absorbant étant déposé suivant un motif défini, et la couche inférieure étant imperméable à la pénétration de particules de matériau absorbant ou de matériau de régulation d'odeur sous la forme de particules ou de petites fibres, ladite couche inférieure ayant une taille de pore moyenne située dans la plage de 5 à 25 µm,
**caractérisée en ce que**
une largeur de ladite bande continue est équivalente à la largeur d'un nombre multiple d'âmes et qu'au moins 70 % des pores de ladite couche inférieure ont un diamètre situé dans la plage de 5 à 15 µm, et que ladite couche inférieure a une perméabilité à l'air inférieure à 1 000 m/s à 196 Pa, et une résistance à la traction dans la direction de la machine d'au moins 20 N/5 cm et une résistance à la traction dans la direction transversale d'au moins 5 N/5 cm, et **en ce que** ladite couche inférieure et ladite couche supérieure sont liées l'une à l'autre par des bandes adhésives.

2. Structure (1) selon la revendication 1, comprenant des âmes individuelles de largeur située dans la plage de 20 à 2 000 mm.

3. Structure (1) selon la revendication 1, dont la couche supérieure (2) est sélectionnée dans le groupe comprenant le tissu tissé, le tissu non tissé, le film synthétique, le papier de soie, le papier airlaid, des composites airlaid et des composites de fibres fines et de filaments continus.

4. Structure (1) selon la revendication 1, dont la couche inférieure comprend un additif hydrophile.

5. Structure (1) selon la revendication 1, comprenant un composite de fibres fines et de filaments continus.

6. Structure (1) selon la revendication 5, comprenant le composite de fibres fines et de filaments continus avec un poids de base de fibres fines dans la plage de 2 à 40 g/m², et un poids de base total de filaments continus dans la plage de 8 à 48 g/m².

7. Structure (1) selon la revendication 5, le composite de fibres fines et de filaments continus comprenant un composite filé-lié/fondu-soufflé.

8. Structure (1) selon la revendication 5, dans laquelle le poids de base des filaments continus est d'environ 4 à 24 g/m² et le poids de base des fibres fines est d'environ 2 à 20 g/m².

9. Structure (1) selon la revendication 5, comprenant une couche filé-lié avec un poids de base dans la plage de 10 à 20 g/m² et une couche fondue-soufflée avec un poids de base dans la plage de 2 à 12 g/m².

10. Structure (1) selon la revendication 5, comprenant une couche filé-lié avec un poids de base dans la plage de 2 à 9 g/m² et une couche fondue-soufflée avec un poids de base dans la plage de 1 à 2 g/m².

11. Structure (1) selon la revendication 5, comprenant au moins une couche filé-lié et au moins une couche fondue-soufflée.

12. Structure (1) selon la revendication 1, comprenant une couche intermédiaire (4) de matériau absorbant comprenant un matériau absorbant sélectionné dans le groupe du matériau gélifiant absorbant, des particules super-absorbantes et des fibres super-absorbantes.

13. Structure (1) selon la revendication 1, comprenant une couche intermédiaire (4) de matériau absorbant comprenant un matériau de régulation d'odeur sélectionné dans le groupe des zéolites, de la silice, de l'argile, du carbone et des composés régulateurs de pH.

14. Structure (1) selon la revendication 1, comprenant une couche supérieure (2) et la couche intermédiaire (4) de matériau absorbant qui sont liées l'une à l'autre thermiquement.

15. Structure (1) selon la revendication 1, dans laquelle la couche inférieure (3) et la couche intermédiaire (4) de matériau absorbant sont liées l'une à l'autre en des points discrets.

16. Structure (1) selon la revendication 15, dans laquelle un polymère thermoplastique activé thermiquement, sélectionné dans le groupe des polyoléfines, du polypropylène, du polyéthylène et de l'acétate de vinyle éthylène, est utilisé pour lier les couches.

17. Structure (1) selon la revendication 1, comprenant plus d'une couche perméable aux liquides et plus d'une couche de matériau absorbant.

18. Structure (1) selon la revendication 1, dans laquelle la couche supérieure (2) comprend des fibres de cellulose avec un poids de base dans la plage de 20 à 1 000 g/m².

19. Structure (1) selon la revendication 1, dans laquelle un matériau de la couche supérieure (2) comprend de 50 à 100 % de cellulose.

20. Structure (1) selon la revendication 1, dans laquelle la couche supérieure (2) comprend au moins 1 % de matériaux synthétiques sélectionnés dans le groupe des polyoléfines, du polypropylène, du polyéthylène, des polyamides, des polyesters et des polyétheresters.

21. Structure (1) selon la revendication 1, dans laquelle le matériau de la couche inférieure (3) est sélectionné dans le groupe des polyoléfines, du polypropylène, du polyéthylène, des polyamides, des polyesters et des polyétheresters.

22. Article absorbant final comprenant une âme absorbante réalisée à partir d'une structure absorbante intermédiaire selon la revendication 1, l'article absorbant comprenant l'âme absorbante avec une barrière intégrée.
